(19) **Europäisches Patentamt**
**European Patent Office**
**Office européen des brevets**

(11) Veröffentlichungsnummer : **0 365 845 B1**

(12) **EUROPÄISCHE PATENTSCHRIFT**

(45) Veröffentlichungstag der Patentschrift :
05.08.92 Patentblatt 92/32

(51) Int. Cl.⁵ : **A61K 7/06,** A61K 7/08

(21) Anmeldenummer : **89117701.6**

(22) Anmeldetag : **25.09.89**

(54) **Verwendung von amphoteren und zwitterionischen Celluloseethern in Haarpflegemitteln.**

Verbunden mit 89910861.7/0437462
(europäische
Anmeldenummer/Veröffentlichungsnummer)
durch Entscheidung vom 23.9.91.

(30) Priorität : **04.10.88 DE 3833658**

(43) Veröffentlichungstag der Anmeldung :
**02.05.90 Patentblatt 90/18**

(45) Bekanntmachung des Hinweises auf die
Patenterteilung :
**05.08.92 Patentblatt 92/32**

(84) Benannte Vertragsstaaten :
**AT BE CH DE ES FR GB GR IT LI NL SE**

(56) Entgegenhaltungen :
DE-A- 3 044 754
DE-A- 3 503 618
FR-A- 2 110 268
FR-A- 2 598 611
CHEMICAL ABSTRACTS, Band 46, Nr. 12, 25.
Juni 1952, Seite 5841, Zusammenfassung Nr.
5842g, Columbus, Ohio, US; & US-A-2591748
(HERCULES POWDER CO.) 08.04.1952 (Kat. D)

(73) Patentinhaber : **Henkel**
**Kommanditgesellschaft auf Aktien**
**Henkelstrasse 67**
**W-4000 Düsseldorf 13 (DE)**

(72) Erfinder : **Müller, Reinhard, Dr.**
**Senliserstrasse 32**
**W-4018 Langenfeld (DE)**
Erfinder : **Hase, Brigitte, Dr.**
**Gerhart-Hauptmann-Strasse 34**
**W-4006 Erkrath 1 (DE)**
Erfinder : **Engelskirchen, Konrad, Dr.**
**Gonellastrasse 24**
**W-4005 Meerbusch 24 (DE)**
Erfinder : **Giede, Karl**
**Schlehenweg 12**
**W-4010 Hilden (DE)**
Erfinder : **Wisotzki, Klaus-Dieter, Dr.**
**Neuendickstrasse 9**
**W-4132 Kamp-Lintfort (DE)**

**Beschreibung**

Die Erfindung betrifft die Verwendung amphoterer und zwitterionischer Celluloseether in Mitteln zur Reinigung und Pflege der Haare sowie Haarpflegemittel, die solche Polymeren als avivierende und kämmbarkeitsverbessernde Zusätze enthalten.

Das Haupthaar weist nach dem Waschen mit Shampoos, Dusch- und Badepräparaten auf Basis von synthetischen Tensiden, vor allem aber nach kosmetischen Behandlungen wie Färben und Verformen, einen kosmetisch unbefriedigenden Zustand auf. Es ist im nassen Zustand nur schwer zu kämmen, fühlt sich im trockenen Zustand stumpf an und sieht glanzlos aus. Es neigt zur statischen Aufladung, wodurch das bekannte "Fliegen" des frisch gewaschenen Haares verursacht wird.

Es ist daher bekannt, nach dem Waschen, Färben oder Dauerwellen des Haares konditionierende Präparate auf das Haar einwirken zu lassen. Als Wirkstoffe in solchen Präparaten sind üblicherweise kationische grenzflächenaktive Stoffe enthalten. Es ist auch bekannt, üblichen Shampoos auf Basis anionaktiver Tenside oder auf Basis von Mischungen von Tensiden unterschiedlicher Ionogenität bestimmte Substanzen beizufügen, um gleichzeitig mit der Haarwäsche einen gewissen konditionierenden Effekt zu erzielen. Solche Substanzen sind z.B. wasserlösliche Proteine oder Proteinabbauprodukte, polykationische Polymere oder auch zwitterionische synthetische Polymerisate, wie sie z.B. in DE OS 21 50 557 oder DE OS 28 17 369 beschrieben sind.

Die bekannten polymeren kationischen Konditionierungswirkstoffe weisen aber zahlreiche Mängel auf. So sind Produkte mit guter Wasserlöslichkeit und ausreichender Verträglichkeit mit anionischen Waschrohstoffen meist zu schwach wirksam, was eine hohe Dosierung erforderlich macht. Andere, begrenzt wasserlösliche Produkte sind zu stark substantiv, sie ziehen nahezu vollständig auf das Haar auf, führen bei mehrmaliger Behandlung des Haares zur Akkumulation und verringern dadurch Fülle und Sitz des getrockneten Haares.

Die für Haarbehandlungs- und Haarfestlegemittel bekannten zwitterionischen Polymeren zeigen, insbesondere in Formulierungen mit anionischen Tensiden, den Nachteil, daß die haaravivierenden und haarfestigenden Eigenschaften im Verlaufe längerer Lagerzeit allmählich verlorengehen.

Es bestand daher die Aufgabe, wasserlösliche Polymere zu finden, die bei Verwendung als Zusatz in wäßrigen Mitteln zur Reinigung und Pflege der Haare, insbesondere in Mitteln zum Waschen und Spülen der Haare, eine gute avivierende, kämmbarkeitsverbessernde Wirkung entfalten und die genannten Nachteile nichtoder in erheblich geringerem Maße - aufweisen.

Gegenstand der Erfindung ist die Verwendung von amphoteren und zwitterionischen, wasserlöslichen Celluloseethern, die durchschnittlich pro Anhydroglucoseeinheit wenigstens 0.1 Amino-und/oder Ammoniumgruppen und wenigstens 0,1 Carboxylgruppen in einer Anordnung der Formeln I und II

$$\left[(CH_2)_m\text{-}^{(+)}NR^1R^2\right]_x R^3 \qquad\qquad -C_nH_{2n}COO^{(-)}$$

$$\text{I} \qquad\qquad\qquad\qquad\qquad\qquad \text{II}$$

worin m = 2-4, n = 1-3, x = 0-3 und $R^1$ und $R^2$ Alkylgruppen mit 1-4 C-Atomen, $R^3$ eine Gruppe $-(CH_2)_m\text{-}NR^1R^2$ oder $-(CH_2)_m\text{-}^{(+)}NR^1R^2R^4$, worin $R^4$ eine Alkylgruppe mit 1-4 C-Atomen oder eine Gruppe $-C_nH_{2n}COO^{(-)}$ ist, sowie gegebenenfalls zusätzlich eine mindestens für ihre Wasserlöslichkeit erforderliche Anzahl etherartig gebundener Alkyl- oder Hydroxyalkylgruppen mit 1-4 C-Atomen enthalten, als kämmbarkeitsverbessernden Zusatz zur Reinigung und Pflege der Haare.

Gruppierungen der Formel I resultieren aus der Reaktion von primär in die Cellulosen eingeführten Dialkylaminoalkylgruppen mit den Aminoalkylierungs- und/oder Carboxylalkylierungsmitteln. Zum Ladungsausgleich können je nach Zusammensetzung der Produkte zusätzliche Kationen und Anionen vorhanden sein, vorzugsweise z.B. Natriumionen oder Protonen und Chlorid- oder Sulfationen.

Vorzugsweise wird allein über die Anzahl der ionischen Gruppen der Formeln I und II die volle Wasserlöslichkeit der Celluloseether eingestellt, und es ist daher bevorzugt, daß sie pro Anhydroglucoseeinheit durchschnittlich insgesamt 0,5 ionische Gruppen in Anordnung der Formeln I und II enthalten. Besonders bevorzugt sind solche Celluloseether, die 0,2 - 0,6 Amino- und/oder Ammoniumgruppen und 0,4 - 0,8 Carboxylgruppen in Anordnung der Formeln I und II enthalten. Dabei wird bevorzugt, daß m = 2, n = 1, $R^1$ und $R^2$ = Ethyl- und $R^3 = (CH_2)_2\text{-}N(C_2H_5)_2$ oder $(CH_2)_2\text{-}^{(+)}N(C_2H_5)_2\,CH_2COO^-$ sind.

Die erfindungsgemäß zu verwendenden Celluloseether sind in Wasser und in wäßrigen Lösungen anionischer, kationischer, ampholytischer, zwitterionischer und nichtionogener Tenside gut löslich und behalten in wäßrigen Lösungen solcher Tenside ihre kämmbarkeitsverbessernden, haaravivierenden und haarfestigenden Eigenschaften auch über längere Lagerzeiten bei. Aufgrund der genannten Eigenschaften eignen sich die erfindungsgemäß zu verwendenden Celluloseether als festigende und haaravivierende Komponente in wäßrigen Zubereitungen zur Reinigung und Pflege der Haare. Solche Zubereitungen können z.B. Haarshampoos, Haarnachspülmittel, Haarfestiger, Haar-Fönwellmittel sowie wäßrige Färbemittel, Dauerwellmittel oder Dauerwell-Fixiermittel sein.

Die erfindungsgemäß zu verwendenden amphoteren Celluloseether ($R^3$ = -$(CH_2)_m$-$NR^1R^2$) sind literaturbekannt bzw. nach literaturbekannten Verfahren herzustellen. Ihre Herstellung wird z. B. in US-PS-2,591,748 beschrieben. Die Herstellung besonders bevorzugter amphoterer Celluloseether wird außerdem in Beispiel 1.1 bis 1.4 näher beschrieben. Die Herstellung erfolgt im wesentlichen dadurch, daß man eine Carboxyalkylcellulose durch Umsetzung mit einer Verbindung der Formel X-$(CH_2)_m$-$NR^1R^2$, worin X = Chlor oder Brom ist und $R^1$ und $R^2$ die für Formel I angegebene Bedeutung haben, alkyliert. Bevorzugt wird Carboxymethylcellulose eingesetzt.

Abweichend von diesem Verfahren kann man aber auch zunächst eine Alkalicellulose mit einer Verbindung der Formel X-$(CH_2)_m$-$NR^1R^2$ und gleichzeitig oder anschließend mit z. B. Natriumchloracetat alkylieren. Dabei werden auch zwitterionische Gruppen der Formel I, in welcher $R^3$ eine Gruppe - $(CH_2)_m$-$^{(+)}NR^1R^2R^4$ und $R^4$ eine Gruppe -$CH_2$-$COO^{(-)}$ ist, gebildet.

Die Einführung von Gruppen der Formel $C_nH_{2n}COO^{(-)}$, worin n = 2 oder 3 ist, wird z.B. durch Anlagerung von Estern bzw. Amiden der Acrylsäure oder Methacrylsäure und anschließende Hydrolyse der Ester- bzw. Amidgruppen erreicht.

Die Einführung der eventuell zur Erreichung der guten Wasserlöslichkeit erforderlichen Alkyl- oder Hydroxyalkylethergruppen mit 1 - 4 C-Atomen erfolgt in üblicher Weise durch Veretherung mit Alkylhalogeniden mit 1 - 4 C-Atomen oder durch Veretherung mit Dialkylsulfat, z. B. Dimethylsulfat, oder durch Anlagerung von Epoxyalkanen wie Ethylenoxid oder Propylenoxid oder Glycid nach literaturbekannten Verfahren. Die Einführung der Alkyl-und Hydroxyalkylgruppen kann vor, gleichzeitig mit oder nach der Einführung der kationischen und/oder anionischen Gruppen erfolgen.

Die erfindungsgemäß zu verwendenden zwitterionischen Polymeren mit quartären Ammoniumgruppen der Formel I (x = 1 - 3, $R^4$ = Alkylgruppen mit 1 - 4 C-Atomen) lassen sich durch Alkylierung der entsprechenden amphoteren Polymeren ($R^3$ = -$(CH_2)_m$-$NR^1R^2$) mit Verbindungen der Formel $R^4$-X und $R^4_2SO_4$ herstellen, wobei $R^4$ eine Alkylgruppe mit 1 - 4 C-Atomen und X = Chlor oder Brom ist, herstellen. Man kann aber auch die Alkalicellulose mit einer Verbindung der Formel X-$(CH_2)_m$-$NR^1R^2$ und anschließend mit Verbindungen der Formeln $R^4$X oder $R^4_2SO_4$ alkylieren und erst dann die -$C_nH_{2n}$-$COO^{(-)}$-Gruppe einführen.

Die erfindungsgemäß zu verwendenden amphoteren und zwitterionischen Celluloseether werden bevorzugt in Mengen von 0,01 - 5 Gew.-% den Mitteln zur Reinigung und Pflege der Haare zugesetzt. Besonders gut wirken die amphoteren und zwitterionischen Celluloseether als Zusätze zu Mitteln zum Waschen oder Spülen der Haare, die durch einen Gehalt an oberflächenaktiven Substanzen, in der Regel in einer Menge von 0,1 - 25 Gew.-% gekennzeichnet sind. Eine bevorzugte Ausführungsform der Erfindung ist z.B. ein Haarshampoo auf wäßriger Basis, das 0,1 - 5 Gew.-% des amphoteren oder zwitterionischen Celluloseethers und 5 - 25 Gew.-% wenigstens eines anionischen Tensids enthält.

Als anionische Tenside eigenen sich in erfindungsgemäßen Haarbehandlungsmitteln alle für die Verwendung am menschlichen Körper geeigneten anionischen oberflächenaktiven Stoffe. Diese sind gekennzeichnet durch eine wasserlöslich machende, anionische Gruppe wie z.B. eine Carboxylat-, Sulfat-, Sulfonat- oder Phosphat-Gruppe und eine lipophile Alkylgruppe mit 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind die Natrium-, Kalium-, Ammonium-, Mono-, Di- und Trialkanolammoniumsalze mit 2 oder 3 C-Atomen in der Alkanolgruppe, von

– linearen Fettsäuren mit 10 bis 22 C-Atomen (Seifen),
– Ethercarbonsäuren der Formel $R^5$-O-$(CH_2$-$CH_2O)_x$-$CH_2$-COOH in der $R^5$ eine lineare Alkylgruppe mit 10 bis 22 C-Atomen und x = O oder 1 bis 12 ist,
– Acylsarcosinen mit 10 bis 18 C-Atomen in der Acylgruppe,
– Acyltauriden mit 10 bis 18 C-Atomen in der Acylgruppe,
– Acylisethionaten mit 10 bis 18 C-Atomen in der Acylgruppe,
– Sulfobernsteinsäuremono- und dialkylestern mit 8 bis 18 C-Atomen in der Alkylgruppe und Sulfobernsteinsäuremonoalkylpolyoxyethylestern mit 8 bis 18 C-Atomen in der Alkylgruppe und 1 bis 6 Oxyethylgruppen,
– linearen Alkansulfonaten mit 12 bis 18 C-Atomen,
– linearen Alpha-Olefinsulfonaten mit 12 bis 18 C-Atomen,
– Alpha-Sulfofettsäuremethylestern von Fettsäuren mit 12 bis 18 C-Atomen,

– Alkylsulfaten und Alkylpolyglykolethersulfaten der Formel $R^6$-O(CH$_2$-CH$_2$O)$_x$-OSO$_3$H, in der $R^6$ eine bevorzugt lineare Alkylgruppe mit 10 bis 18 C-Atomen und x = O oder 1 bis 12 ist.

Bevorzugt sind Alkylsulfate- und Alkylpolyglykolethersulfate mit 10 bis 18 C-Atomen in der Alkylgruppe und bis zu 12 Glykolethergruppen im Molekül.

Neben den genannten anionischen Tensiden können die erfindungsgemäßen Haarshampooformulierungen alle für diesen Zweck bekannten Hilfs- und Zusatzmittel in den dafür üblichen Mengen enthalten. Dies sind insbesondere nichtionogene, amphotere und zwitterionische Tenside.

Nichtionische Tenside sind vor allem die Anlagerungsprodukte von 2 bis 20 Mol Ethylenoxid an bevorzugt lineare Alkohole mit 12 bis 18 C-Atomen, an Alkylphenole mit 8 bis 15 C-Atomen in der Alkylgruppe, an Fettsäuren mit 12 bis 18 C-Atomen, an Fettsäurepartialglyceride, an Fettsäure-sorbitan-partialester, an Fettsäurealkanolamide und an Methylglucosid-Fettsäureester. Weitere geeignete nichtionogene Tenside sind Alkyl(oligo)-glucoside, Alkylaminoxid-Tenside und Fettsäurealkanolamide. Amphotere Tenside sind z.B. Alkyl(C$_8$-C$_{18}$)-trimethylammonio-glycinat oder Acyl-(C$_8$-C$_{18}$)-aminopropyltrimethylammonio-glycinat.

Eine besonders bevorzugte Ausführungsform der Erfindung sind Mittel zum Nachspülen der Haare, z.B. nach einer Shampoobehandlung oder auch nach einer Färbe- oder Bleichbehandlung. Solche Produkte enthalten in der Regel 0,1 - 10 Gew.-% eines kationischen Tensids zur Verringerung der antistatischen Aufladbarkeit der Haare. Durch den erfindungsgemäßen Zusatz von 0,1 - 1 Gew.-% eines amphoteren oder zwitterionischen wasserlöslichen Celluloseethers wird die Kämmbarkeit des Haars und die Fülle der trockenen Frisur weiter verbessert. Besonders zweckmäßig ist ein Gehalt von 0,5 - 5 Gew.-% eines kationischen Tensids der allgemeinen Formel IV

$$(IV) \qquad \left[ R^7 - \overset{\displaystyle R^8}{\underset{\displaystyle R^9}{N^{(+)}}} - R^{10} \right] \qquad A^{(-)}$$

In der $R^7$ eine Alkyl- oder Hydroxyalkylgruppe mit 8 - 22 C-Atomen, eine Gruppe $R^{11}$-CONH(CH$_2$)$_y$-, in der $R^{11}$ eine Alkylgruppe mit 7 - 21 C-Atomen und y eine Zahl von 2 - 4 ist, $R^8$ und $R^9$ eine Alkylgruppe mit 1 - 4 C-Atomen oder eine Gruppe der Formel -(C$_m$H$_{2m}$O)$_z$H, in der m eine Zahl von 2 - 4 und z eine Zahl von 1 - 10 ist und $R^{10}$ eine Benzylgruppe ist oder eine der für $R^7$, $R^8$ und $R^9$ angegebenen Bedeutungen hat, und $A^{(-)}$ ein Chlorid-, Bromid-, Hydrogensulfat, Hydrogenphosphat, Methoxysulfat oder Ethoxysulfatanion ist.

Geeignete kationische Tenside sind z.B. Cetyltrimethylammoniumchlorid, Lauryldimethylbenzylammoniumchlorid, Stearyltrimethylammoniumchlorid, Distearyldimethylammoniumchlorid, Talgalkyltris-(oligooxyethyl)-ammonium-phospat, 2-Hydroxyhexadecyl-2-hydroxyethyl-dimethylammoniumchlorid und 2-Hydroxyhexadecyl-bis-(2-hydroxyethyl)-methylammoniumchlorid.

Zur Verbesserung der haaravivierenden Wirkung ist es weiterhin zweckmäßig, daß die erfindungsgemäßen Haarspülungen zusätzlich einen Fettstoff, z.B. eine kosmetische Öl-, Fett- oder Wachskomponente, ein Paraffinöl- oder Paraffinwachs, Wollwachs oder ein Wollwachsderivat enthalten. Als Fettstoff sind besonders Fettalkohole mit 14 - 22 C-Atomen, z.B. Cetyl- und/oder Stearylalkohol sowie Fettsäuremonoglyceride und/oder Fettsäurediglyceride von Fettsäuren mit 16 - 22 C-Atomen, z. B. von Palmitin und/oder Stearinsäure geeignet. Solche Fettstoffe sind bevorzugt in Mengen von insgesamt 1 - 10 Gew.-%, bezogen auf die gesamte Zubereitung enthalten.

Außer den genannten Komponenten können die erfindungsgemäßen Haarspülmittel noch weitere, an sich in Haarspülmitteln übliche Hilfs- und Zusatzmittel in untergeordneten Mengen bis etwa 5 Gew.-% enthalten. Solche Hilfs- und Zusatzmittel sind z. B. niedere Polyole, 1.2-Propylenglykol oder Glycerin, wasserlösliche nichtionogene Verdickungsmittel wie z. B. Hydroxyethylcellulose, Methylhydroxypropylcellulose, Hydroxyethylstärke und Hydroxypropylguar, weiterhin Konservierungsstoffe, Duftstoffe, Farbstoffe, Lichtschutzmittel sowie haarkosmetische Wirkstoffe wie z.B. Vitamine, Pflanzenextrakte, Balsame, Antischuppenwirkstoffe (z.B. Zn- oder Mg-Pyridinthion) oder Sebostatika.

Die folgenden Beispiele sollen den Erfindungsgegenstand näher erläutern. Eingetragene Wavenzeichen und Handelsbezeichwingen werden als solche anerkannt.

<u>BEISPIELE</u>

1. Herstellungsbeispiele amphoterer Celluloseether

Beispiel 1.1

688 g Aceton (Suspensionsmittel) wurden in einem Rührbecher vorgelegt und darin nach Einleiten von Stickstoff 95,5 g (0,4 Mol Anhydroglucoseeinheiten) Natrium-Carboxymethylcellulose (CMC, Viskosität 1 %ig in Wasser: 1250 mPas (20 °C), Carboxymethylierungsgrad: $DS_C = 0,73$, Wassergehalt 7,6 Gew.-%) unter Rühren mit einem Pendraulikrührer suspendiert. Nach fünf Minuten Rühren unter Stickstoff wurden innerhalb von fünf Minuten 40 g (0,5 mol) 50 %ige wäßrige Natronlauge zugetropft. Nach weiteren fünf Minuten wurden 86 g (0,25 mol) einer 50 %igen wäßrigen 2-Chlorethyldiethylaminhydrochloridlösung (CDA) zugetropft. Nach fünf Minuten Rühren wurde das Gemisch in einen 2-l-Kolben übergeführt und unter Überleiten von Stickstoff vier Stunden zum Rückfluß erhitzt. Das Rohprodukt wurde abgesaugt und mit 80 %igem wäßrigen Aceton gewaschen, bis kein Chlorid mehr nachweisbar war. Die Ausbeute betrug 111 g. Das Produkt hatte folgende Kennzahl:
Substitutionsgrad bezogen auf stickstoffhaltige Gruppen ($MS_N$): 0,39 (ermittelt durch Stickstoffbestimmung)

Beispiel 1.2

Nach dem unter 1.1 beschriebenen Verfahren wurde ein weiteres Produkt hergestellt:
Ausgangsprodukt: CMC, Viskosität 1 %ig in Wasser : 550 mPas (20 °C), $DS_C = 0,70$
Molverhältnis CMC : NaOH : CDA = 1 : 1 : 0,50
Das Reaktionsprodukt hatte folgende Kennzahl:
$MS_N$ : 0,21

Beispiel 1.3

Nach dem unter 1 beschriebenen Verfahren wurde ein weiteres Produkt hergestellt.
Ausgangsprodukt: CMC, Viskosität 1 %ig in Wasser : 2000 mPas (20 °C), $DS_C$ : 0,92
Molverhältnis CMC : NaOH : CDA = 1 : 1,25 : 0,625
Das Reaktionsprodukt hatte folgende Kennzahlen:
$MS_N$ : 0,22

Beispiel 1.4

516 g Aceton wurden in einem Rührbecher vorgelegt und darin nach Einleiten von Stickstoff 34,4 g (0,2 mol) Cellulosepulver (Wassergehalt 5,8 %) unter Rühren mit einem Pendraulikrührer suspendiert. Nach fünf Minuten Rühren unter Stickstoff wurden innerhalb von fünf Minuten 32 g (0,4 mol) 50%ige wäßrige Natronlauge zugetropft. Nach weiteren fünf Minuten wurden 68,8 g (0,2 mol) einer 50%igen wäßrigen 2-Chlorethyldiethylaminhydrochloridlösung zugetropft. Nach fünf Minuten Rühren wurde das Gemisch in einen 2-l-Kolben übergeführt und unter Überleiten von Stickstoff eine Stunde zum Rückfluß erhitzt. Danach wurden 36 g (0,45 mol) 50%ige wäßrige Natronlauge und im Anschluß 26,6 g (0,225 mol) 80%ige wäßrige Chloressigsäurelösung zugetropft und das Reaktionsgemisch unter Rühren unter Stickstoff weitere drei Stunden zum Rückfluß erhitzt. Das Rohprodukt wurde abgesaugt und mit 80%igem wäßrigen Aceton gewaschen, bis kein Chlorid mehr nachweisbar war. Die Ausbeute betrug 54,1 g. Das Produkt hatte folgende Kennzahlen:
$MS_N$ : 0,56
$DS_C$ : 0,86
Wassergehalt : 6,4 Gew.-%.

2. Anwendungsbeispiele

2.1 Shampoos

Beispiel 2.1.1

| | |
|---|---|
| Texapon ALS[1] | 33,3 Gew.-% |
| Dehyton K[2] | 16,7 Gew.-% |
| Celluloseether aus | |
| Beispiel 1.1 | 0,5 Gew.-% |
| Kochsalz | 0,5 Gew.-% |
| Wasser | 49,0 Gew.-% |

Beispiel 2.1.2

| | |
|---|---|
| Texapon N 25[3] | 21,5 Gew.-% |
| Sulfosuccinat S 2[4] | 30,0 Gew.-% |
| Celluloseether aus | |
| Beispiel 1.2 | 0,5 Gew.-% |
| Kochsalz | 0,5 Gew.-% |
| Wasser | 47,5 Gew.-% |

Beispiel 2.1.3

| | |
|---|---|
| Texapon N 25[3] | 35,7 Gew.-% |
| Akypo Soft KA 250 BV[5] | 16,7 Gew.-% |
| Celluloseether aus | |
| Beispiel 1.3 | 0,5 Gew.-% |
| Kochsalz | 0,5 Gew.-% |
| Wasser | 46,6 Gew.-% |

Beispiel 2.1.4

| | |
|---|---|
| Texapon N 25[3] | 26,8 Gew.-% |
| Alkylglucosid (Hl-Be 94-0)[6] | 12,3 Gew.-% |
| Celluloseether aus | |
| Beispiel 1.1 | 0,5 Gew.-% |
| Kochsalz | 0,5 Gew.-% |
| Wasser | 59,9 Gew.-% |

Beispiel 2.1.5

| | |
|---|---|
| Texapon N 25[3] | 21,4 Gew.-% |
| Comperlan KD[7] | 6,0 Gew.-% |
| Celluloseether aus | |
| Beispiel 1.2 | 0,5 Gew.-% |
| Kochsalz | 0,5 Gew.-% |
| Wasser | 71,6 Gew.-% |

Beispiel 2.1.6

| | |
|---|---|
| Texapon NSW[8] | 56,0 Gew.-% |
| Dehyton K[2] | 10,0 Gew.-% |
| Celluloseether aus | |
| Beispiel 1.4 | 0,5 Gew.-% |
| Kochsalz | 0,5 Gew.-% |
| Wasser | 33,0 Gew.-% |

Das mit den aufgeführten Shampoos gewaschene Haar zeigt eine gute Naßkämmbarkeit und Trockenkämmbarkeit sowie keine elektrostatische Aufladung beim Kämmen nach dem Trocknen.

2.2 Spülungen

Beispiel 2.2.1

| | |
|---|---|
| Dehyquart A[9] | 3,0 Gew.-% |
| Stenol 1618[10] | 3,0 Gew.-% |
| Culminal MHPC 3000[11] | 0,6 Gew.-% |
| Celluloseether aus | |
| Beispiel 1.1 | 0,5 Gew.-% |
| Wasser | 92,9 Gew.-% |

Beispiel 2.2.2

| | |
|---|---|
| Dehyquart A[9] | 3,0 Gew.-% |
| Stenol 1618[10] | 3,0 Gew.-% |
| Culminal MHPC 3000[11] | 0,6 Gew.-% |
| Genamin DSAC[12] | 1,0 Gew.-% |
| Celluloseether aus | |
| Beispiel 1.2 | 0,5 Gew.-% |
| Wasser | 91,9 Gew.-% |

Beispiel 2.2.3

| | |
|---|---|
| Dehyquart E[13] | 3,0 Gew.-% |
| Stenol 1618[10] | 3,0 Gew.-% |
| Culminal MHPC 3000[11] | 0,6 Gew.-% |
| Genamin DSAC[12] | 1,0 Gew.-% |
| Celluloseether aus Beispiel 1.3 | 0,5 Gew.-% |
| Wasser | 91,9 Gew.-% |

Beispiel 2.2.4

| | |
|---|---|
| Dehyquart SP[14] | 3,0 Gew.-% |
| Stenol 1618[10] | 3,0 Gew.-% |
| Culminal MHPC 3000[11] | 0,6 Gew.-% |
| Genamin DSAC[12] | 1,0 Gew.-% |
| Celluloseether aus Beispiel 1.4 | 0,5 Gew.-% |
| Wasser | 91,9 Gew.-% |

Die aufgeführten Nachspülmittel verleihen dem nassen und trockenen Haar eine sehr gute Kämmbarkeit und einen angenehmen Griff. Beim Kämmen des trockenen Haares mit einem Hartgummikamm tritt keine elektrostatischen Aufladung ein.

1 Ammoniumlaurylsulfat (30 % AS)
2 Kokosalkylamidopropyl-dimethyl-glycin (30 % AS)
3 Fettalkohol-$C_{12-14}$ + 2EO-sulfat, Na-Salz (28 % AS)
4 Fettalkohol-$C_{12-14}$ + 2EO-sulfobernsteinsäuremonoester, Di-Na-Salz (30 %)
5 Kokosfettsäuremonoethanolamid-4-carboxylat, Na-Salz (Ethercarbonsäure) (30 % AS)
6 $R_1O(Z)_x$ mit Z = Glucose, x = 1,4 und $R_1$ = n-Alkyl ($C_{12-14}$) (60,8 % AS)
7 Kokosfettsäurediethanolamid
8 Fettalkohol-$C_{12-14}$ + 2EO-sulfat, Na-Salz (28 % AS)
9 Cetyltrimethylammoniumchlord (25 % AS)
10 Fettalkohol-$C_{16-18}$
11 Methylhydroxypropylcellulose (eingesetzt als 3 %ige Lösung in Wasser)
12 Dimethyldistearylammoniumchlorid
13 N-(2-Hydroxyhexadecyl-1)-N,N-dimethyl-N-2-hydroxyethylammoniumchlorid (28 % AS)
14 $(H-(O-CH_2-CH_2-)_{10})_3(CH_3-(CH_2)_{14-16}-CH_2-)N^+ H_2PO_4^-$(50 % AS) (% AS bedeutet: Gew.-% gelöst in Wasser)

**Patentansprüche**

1. Verwendung von amphoteren und zwitterionischen, wasserlöslichen Celluloseethern, die durchschnittlich pro Anhydroglucoseeinheit wenigstens 0,1 Amino- und/oder Ammoniumgruppen und wenigstens 0,1 Carboxylgruppen in einer Anordnung der Formeln I und II

EP 0 365 845 B1

$$\left.-\left[(CH_2)_m-^{(+)}NR^1R^2\right| R^3\right]_x \qquad -C_nH_{2n}COO^{(-)}$$

I II

worin m = 2-4, n = 1-3, x = 0-3 und $R^1$ und $R^2$ Alkylgruppen mit 1-4 C-Atomen, $R^3$ eine Gruppe $-(CH_2)_m-NR^1R^2$ oder $-(CH_2)_m-^{(+)}NR^1R^2R^4$, worin $R^4$ eine Alkylgruppe mit 1-4 C-Atomen oder eine Gruppe $-C_nH_{2n}COO^{(-)}$ ist, sowie gegebenenfalls zusätzlich eine mindestens für ihre Wasserlöslichkeit erforderliche Anzahl etherartig gebundener Alkyl- oder Hydroxyalkylgruppen mit 1-4 C-Atomen enthalten, als kämmbarkeitsverbessernden Zusatz zur Reinigung und Pflege der Haare.

2. Verwendung nach Anspruch 1, dadurch gekennzeichnet, daß die Celluloseether pro Anhydroglucoseeinheit insgesamt mindestens 0,5 ionische Gruppen in der Anordnung gemäß Formel I und II enthalten.

3. Verwendung nach Anspruch 1, dadurch gekennzeichnet, daß die Celluloseether pro Anhydroglucoseeinheit durchschnittlich 0,2-0,6 Amino- und/oder Ammoniumgruppen und 0,4-0,8 Carboxylgruppen in der Anordnung gemäß Formel I und II, mit m = 2, n = 1, $R^1$ und $R^2$ = $C_2H_5$ und $R^3$ = $(CH_2)_2-N(C_2H_5)_2$ bzw. $(CH_2)_2-^{(+)}N(C_2H_5)_2CH_2COO^{(-)}$, enthalten.

4. Verwendung nach einem der Ansprüche 1 oder 2, dadurch gekennzeichnet, daß die Mittel zur Reinigung und Pflege der Haare 0,01 - 5 Gew.-% der amphoteren oder zwitterionischen Celluloseether und 0,1 - 25 Gew.- einer oberflächenaktiven Substanz enthalten.

5. Mittel zum Nachspülen der Haare, dadurch gekennzeichnet, daß es 0,1 - 10 Gew.% eines kationischen Tensids und 0,1 - 1 Gew.-% eines amphoteren oder zwitterionischen, wasserlöslichen Celluloseethers gemäß Anspruch 1 enthält.

6. Mittel zum Nachspülen der Haare gemäß Anspruch 5, dadurch gekennzeichnet, daß es zusätzlich 1 - 10 Gew.-% eines Fettstoffes aus der Gruppe Fettalkohole mit 14 - 22 C-Atomen oder Fettsäuremonoglyceride und/oder Fettsäurediglyceride von Fettsäuren mit 16 - 22 C-Atomen enthält.

## Claims

1. A hair treating composition comprising an amount effective to reduce the tendency of hair toward electrostatic charging of amphoteric and zwitterionic water soluble substituted cellulose ether molecules, said cellulose ether molecules being substituted with an average, per anhydroglucose unit, of at least 0.1 amino or ammonium groups and at least 0.1 carboxylate groups corresponding to formula I and II respectively:

$$\left[(CH_2)_m-^{(+)}NR^1R^2\right]R^3_x \qquad -C_nH_{2n}COO^-$$

I II,

wherein m is an integer from 2 to 4 inclusive, n is an integer from 1 to 3 inclusive, x is an integer from 0 to 3 inclusive, each of $R^1$ and $R^2$ is a $C_{1-4}$ alkyl group, and $R^3$ is a group having one of the formulas: $-(CH_2)m-NR^1R^2$ or $-(CH_2)_m-^+N R^1R^2R^4$, where $R^4$ is a $C_{1-4}$ alkyl group or a group having the formula $-C_nH_2nCOO-$, said substituted cellulose ether molecules also bearing, if necessary for full solubility in water, sufficient $C_{1-4}$ alkyl, $C_{1-4}$ hydroxyalkyl, or mixed alkyl and hydroxylalkyl ether substituents.

2. A composition as claimed in claim 1, wherein the cellulose ether molecules contain on average, per anhydroglucoside unit, at least 0.5 ionic groups corresponding to one of the formulas I and II.

3. A composition as claimed in claim 2, wherein the cellulose ether molecules contain on average, per anhydroglucose unit, (i) 0.2 to 0.6 groups corresponding to formula I when m = 2, n = 1, each of $R^1$ and $R^2$ = $C_2H_5$, and $R^3$ = $(CH_2)_2-N(C_2H_5)_2$ or $(CH_2)_2-^{(+)}N(C_2H_5)_2CH_2COO^-$ and (ii) 0.4 to 0.8 groups corresponding to formula 11 when n = 1.

4. A composition as claimed in claim 1, 2, or 3, comprising from 0.01 to 5% by weight of the amphoteric zwitterionic substituted cellulose ethers and from 0.1 to 25% by weight of a surfactant.

5. A hair rinsing composition, comprising from 0.1 to 10% by weight of a cationic surfactant and from 0.1

9

to 1% by weight of an amphoteric zwitterionic water soluble substituted cellulose ether of the type claimed in claim 1.

6. A hair rinsing composition as claimed in claim 5, additionally comprising from 1 to 10% by weight of a fatting agent seected from the group consisting of (i) $C_{14-22}$ aliphatic alcohols and (ii) aliphatic acid monoglycerides and aliphatic acid diglycerides of $C_{16-22}$ aliphatic acids.

## Revendications

1. Utilisation d'éthers cellulosiques hydrosolubles amphotères et zwitterioniques qui contiennent en moyenne par unité d'anhydroglucose au moins 0,1 groupe amino et/ou ammonium et au moins 0,1 groupe carboxyle dans la disposition donnée dans les formules I et II

$$-\left[ (CH_2)_m - {}^{(+)}NR^1R^2 \right]_X \quad R3 \qquad\qquad -C_nH_{2n}COO^{(-)}$$

$$I \qquad\qquad\qquad\qquad II$$

dans lesquelles m = 2-4, n = 1-3, x = 0-3 et $R^1$ et $R^2$ représentent des groupes alkyle contenant de 1 à 4 atomes de carbone, $R^3$ représente un groupe $-(CH_2)_m-NR^1R^2$ ou $-(CH_2)_m-{}^{(+)}NR^1R^2R^4$, où $R^4$ représente un groupe alkyle contenant de 1 à 4 atomes de carbone, ou encore un groupe $-C_2H_{2n}COO^{(-)}$, ainsi qu'éventuellement, en outre, un nombre au moins requis pour leur hydrosolubilité, de groupes alkyle ou hydroxyalkyle liés pour former des groupes éthers et contenant de 1 à 4 atomes de carbone, comme additif rendant les cheveux plus aptes à être peignés, pour le nettoyage et les soins des cheveux.

2. Utilisation selon la revendication 1, caractérisée en ce que les éthers cellulosiques contiennent par unité d'anhydroglucose au total au moins 0,5 groupe ionique dans la disposition selon les formules I et II.

3. Utilisation selon la revendication 1, caractérisée en ce que les éthers cellulosiques contiennent par unité d'anhydroglucose en moyenne de 0,2 à 0,6 groupe amino et/ou ammonium et de 0,4 à 0,8 groupe carboxyle dans la disposition des formules I et II, avec m = 2, n = 1, $R^1$ et $R^2$ = $C_2H_5$ et $R^3$ = $(CH_2)_2-N(C_2H_5)_2$ ou $(CH_2)_2-{}^{(+)}N(C_2H_5)_2CH_2COO^{(-)}$.

4. Utilisation selon l'une quelconque des revendications 1 ou 2, caractérisée en ce que les agents destinés au nettoyage et aux soins des cheveux contiennent de 0,01 à 5% en poids des éthers cellulosiques amphotères ou zwitterioniques et de 0,1 à 25% en poids d'un substance tensioactive.

5. Agent pour le rinçage ultérieur des cheveux, caractérisé en ce qu'il contient de 0, 1 à 10% en poids d'un agent tensioactif cationique et de 0,1 à 1% en poids d'un éther cellulosique hydrosoluble amphotère ou zwitterionique selon la revendication 1.

6. Agent pour le rinçage ultérieur des cheveux selon la revendication 5, caractérisé en ce qu'il contient, en outre, de 1 à 10% en poids d'une matière grasse choisie parmi le groupe des alcools gras contenant de 14 à 22 atomes de carbone ou encore des monoglycérides d'acides gras et/ou des diglycérides d'acides gras contenant de 16 à 22 atomes de carbone.